# EUROPÄISCHE PATENTANMELDUNG

(11) **EP 1 401 241 A2**
(43) Veröffentlichungstag der Anmeldung: **24.03.2004**
(21) Anmeldenummer: 03020705.4
(22) Anmeldetag: 11.09.2003
(51) Int. Cl.: H04R 25/00, A61B 5/12

(54) **Schnittstellenvorrichtung für audiologische Geräte und entsprechendes Verfahren zum Datenaustausch**

(30) Priorität: 23.09.2002 DE 10244136
(71) Anmelder: Siemens Audiologische Technik GmbH, 91058 Erlangen (DE)
(72) Erfinder: Aschoff, Stefan, 91088 Bubenreuth (DE); Bindner, Jörg, 91085 Weisendorf (DE); Riecken, Volker, 91052 Erlangen (DE); Weidner, Roland, 96199 Zapfendorf (DE)
(74) Vertreter: Berg, Peter, Dipl.-Ing.

(57) **Zusammenfassung**

Die Schnittstelle zwischen den Applikationen (11, 14, 15) verschiedener audiologischer Geräte zu verschiedenen Datenverwaltungssystemen (71, 72, 73) soll verbessert werden. Daher ist erfindungsgemäß eine universelle Schnittstelle (69) vorgeschlagen, die über ein uniformes Interface für die Applikationen und mehrere Interfaces für die verschiedenen Datenverwaltungssysteme verfügt. Daneben sind die entsprechenden Konvertierroutinen vorgesehen. Darüber hinaus kann ein Klassenframework in dem Interface (69) vorgesehen sein, die jede Applikation verwenden kann, so dass sie einen einheitlichen Zugriff auf das universelle Interface (69) erhält.

## Beschreibung

Die vorliegende Erfindung betrifft eine Schnittstellenvorrichtung für audiologische Geräte. Darüber hinaus betrifft die vorliegende Erfindung ein entsprechendes Verfahren zum Datenaustausch für audiologische Geräte.

Für die Einstellung eines Hörgeräts durch den Akustiker oder Arzt sind in der Regel mehrere audiologische Geräte notwendig. So wird beispielsweise mit einem Audiometer der Hörschaden eines Patienten vermessen, während mit einem Programmiergerät ein geeignetes Hörgerät entsprechend den audiometrischen Daten programmiert wird. Ferner kann das Hörgerät in einer Testbox auf Funktionsfähigkeit überprüft werden. Jedes dieser Geräte besitzt eine Applikation und somit ein proprietäres Format für Dateneingang und Datenausgang.

Die Mess- beziehungsweise Steuerdaten der einzelnen Geräte werden in einem oder mehreren Datenverwaltungssystemen verwaltet beziehungsweise abgelegt. Für die Kommunikation einer Applikation mit einem Datenverwaltungssystem ist eine Schnittstelle erforderlich. Für die Kommunikation zweier Applikationen, die über ein Datenverwaltungssystem läuft, sind somit zwei Interfaces beziehungsweise Schnittstellen erforderlich. Bei einem Geflecht von mehreren Applikationen und mehreren Datenverwaltungssystemen sind folglich ebenso viele Schnittstellen notwendig, wie es Zugriffsmöglichkeiten von Applikationen auf Datenverwaltungssysteme gibt. Dabei werden mit unterschiedlichen Applikationen mittels unterschiedlicher Interface-Technologien aus verschiedenen Datenverwaltungssystemen dieselben Daten abgegriffen beziehungsweise ausgetauscht. Dies wird bislang stets mit Interface-Modulen bewerkstelligt, die individuell für eine Kombination Applikation/Interface-Technologie/Datenverwaltungssystem/Datentyp erstellt wurden. Ein Nachteil derartiger Systeme ist insbesondere ihre Fehleranfälligkeit und die aufwändige Implementation. Darüber hinaus sind die unterschiedlichen Interfaces vielfach mit ähnlichen Routinen befüllt.

Die Aufgabe der vorliegenden Erfindung besteht darin, die Schnittstellen zwischen den verschiedenen Applikationen audiologischer Geräte und den entsprechenden Datenverwaltungssystemen zu verbessern.

Erfindungsgemäß wird diese Aufgabe gelöst durch eine Schnittstellenvorrichtung für audiologische Geräte zwischen mehreren Applikationen einerseits und mindestens einem Datenverwaltungssystem andererseits mit einer Applikationsanschlusseinrichtung, an die die mehreren Applikationen zum uniformen Datenaustausch anschließbar sind, einer Datenverwaltungsanschlusseinrichtung, an die das mindestes eine Datenverwaltungssystem zum Datenaustausch anschließbar ist, und einer Konvertiereinrichtung, die zwischen die Applikationsanschlusseinrichtung und die Datenverwaltungsanschlusseinrichtung geschlossen ist, zum Konvertieren von von der Applikationsanschlusseinrichtung aufgenommenen für die mehreren Applikationen jeweils spezifischen Applikationsdaten in ein vorgebbares Datenbankformat und/oder zum Konvertieren von von der Datenverwaltungsanschlusseinrichtung aufgenommenen Datenbankdaten in ein oder mehrere für die mehreren Applikationen jeweils spezifischen Applikationsformate.

Darüber hinaus wird die oben genannte Aufgabe gemäß der vorliegenden Erfindung gelöst durch ein Verfahren zum Datenaustausch für audiologische Geräte zwischen mehreren Applikationen einerseits und mindestens einem Datenverwaltungssystem andererseits durch uniformen Datenaustausch von Applikationsdaten mit mehreren der Applikationen durch eine Schnittstellenvorrichtung, Datenaustausch von Datenbankdaten mit dem mindestens einen Datenverwaltungssystem durch die Schnittstellenvorrichtung, und Konvertieren der für die mehreren Applikationsdaten jeweils spezifischen Applikationsdaten in ein vorgebbares Datenbankformat für das mindestens eine Datenverwaltungssystem und/oder Konvertieren von Datenbankdaten in ein oder mehrere für die mehreren Applikationen jeweils spezifischen Applikationsformate.

Die vorliegende Erfindung ermöglicht somit in vorteilhafter Weise, dass mit Hilfe eines einzigen Interfaces ein Datenaustausch zwischen mehreren Applikationen und mehreren Datenverwaltungssystemen für eine Vielzahl unterschiedlicher Daten möglich ist. Dadurch sinkt die Fehleranfälligkeit beträchtlich und der Implementierungsaufwand reduziert sich enorm.

Die erfindungsgemäße Schnittstellenvorrichtung kann mit einer Klassenbibliothek, d.h. Klassenframework, auf die mit jeder der mehreren Applikationen zugreifbar ist, ausgestattet sein. Damit können die einzelnen Applikationen, die Zugriff auf die Klassenbibliothek haben, für bestimmte Klassen Objekte instantiieren, so dass ein uniformes Interface für die Applikationen entwickelt werden kann.

In vorteilhafter Weise ist eine Zustandsverwaltungseinrichtung für die mehreren Applikationen vorgesehen, so dass die mehreren Applikationen auf vorgegebene Daten gemeinsam Zugriff haben (state sharing). Hierdurch können sich zwei oder mehr Applikationen einen Zustand, d.h. Daten, teilen.

Darüber hinaus können mit der Zustandsverwaltungseinrichtung Zustände und Daten der mehreren Applikationen in einer Datenbank zum gemeinsamen Zugriff abgespeichert werden (state and data notifying). Dadurch kann die Redundanz der Datenhaltung reduziert werden.

Vorzugsweise kann mit der Zustandsverwaltungseinrichtung automatisch erkannt werden, welches oder welche Datenverwaltungssysteme an das Interface angeschlossen sind. Damit wird den Applikationen die Aufgabe abgenommen, festzustellen, in welchem Datenverwaltungsumfeld sie sich befinden.

Günstigerweise ist in dem Interface, d.h. in der Schnittstellenvorrichtung, eine Datenhaltungseinrichtung, insbesondere ein flüchtiger Speicher, zum Halten von Daten für mehrere Applikationen vorgesehen. Entsprechende Datenhaltungsalgorithmen bieten die Möglichkeit, Daten dort abzulegen, wo sie am nächsten zu einem Datenverwaltungssystem sind. Dort findet der gesamte Abgleich und die Synchronisation über Applikationen hinweg statt.

Eine mit diesen Merkmalen ausgestattete Schnittstellenvorrichtung bietet die Möglichkeit mit einem einzigen Interface Daten in unterschiedliche Datenverwaltungssysteme zu speichern und untereinander auszutauschen.

Die vorliegende Erfindung wird nun anhand der beigefügten Zeichnungen näher erläutert, in denen zeigen:
- FIG 1: ein Blockschaltdiagramm der Hardwarekomponenten eines audiologischen Messsystems;
- FIG 2: ein Blockschaltdiagramm eines Datensystems gemäß dem Stand der Technik;
- FIG 3: ein Blockschaltdiagramm eines audiologischen Datensystems gemäß der vorliegenden Erfindung.

Die nachfolgenden Ausführungsbeispiele sind bevorzugte Ausführungsformen der vorliegenden Erfindung.

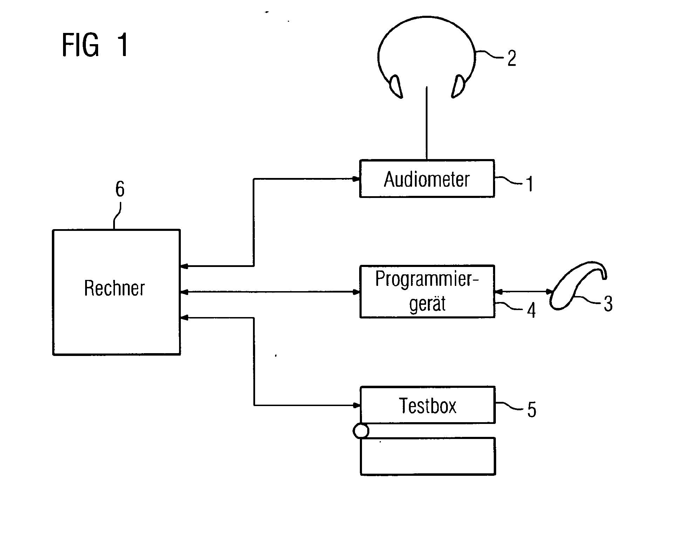

In FIG 1 ist ein Blockschaltbild eines typischen Hardwareaufbaus der Instrumente eines Akustikers dargestellt. Mit einem Audiometer 1, an das Kopfhörer 2 angeschlossen sind, wird das Gehör eines Patienten vermessen. Ein Hörgerät 3 wird von einem Programmiergerät 4 programmiert. Nach dem Programmieren wird das Hörgerät 3 in einer Testbox auf Funktionsfähigkeit überprüft. Jedes der Geräte 1, 4 und 5 verfügt über eine Software-Applikation zur Steuerung des Geräts und zum Datenaustausch. Die Geräte sind mit einem Rechner 6 verbunden, der die erfindungsgemäße uniforme Schnittstelle (nicht eingezeichnet) und entsprechende Datenverwaltungssysteme (ebenfalls nicht eingezeichnet) bereitstellt.

Über die gemeinsame Schnittstelle (Interface) können die einzelnen Geräte untereinander oder aber mit einem oder mehreren Datenverwaltungssystemen kommunizieren. Die Datenverwaltungssysteme, die in der Regel Datenbanken beinhalten, können in den Rechner 6 integriert oder aber über entsprechende Datennetze zur Verfügung gestellt werden.

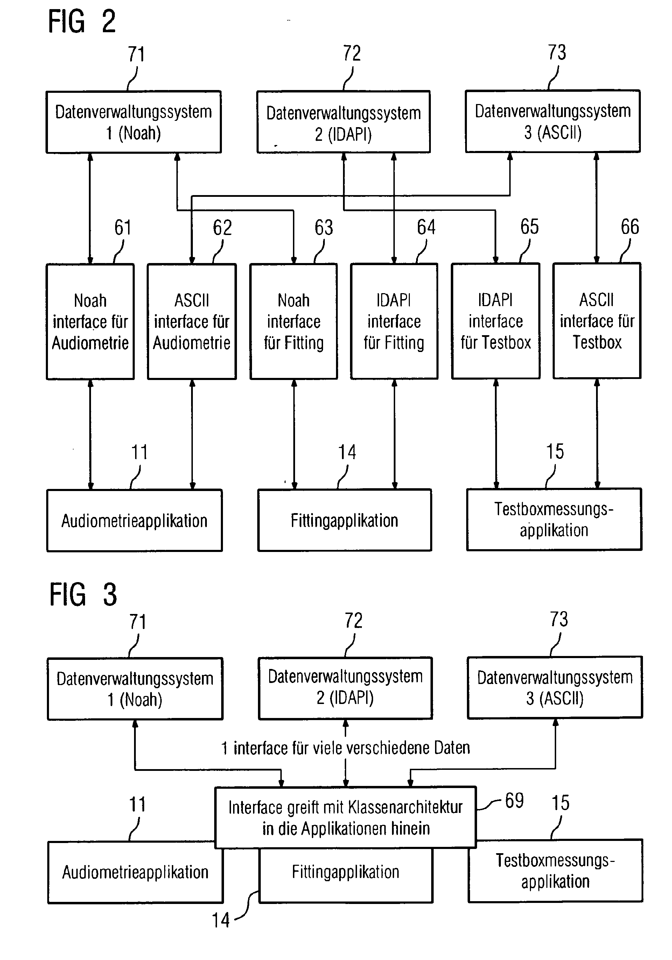

Zur Verdeutlichung der Erfindung sei zunächst in FIG 2 ein Blockschaltbild eines Datensystems nach dem Stand der Technik betrachtet. Entsprechend dem Beispiel von FIG 1 wird eine Audiometrieapplikation 11 für das Audiometer 1, eine Fittingapplikation 14 für das Programmiergerät 4 und eine Testboxmessungsapplikation 15 für die Testbox 5 zur Qualitätssicherung bereitgestellt. In einem Rechner 6 gemäß dem Stand der Technik wird eine Vielzahl von Interfaces 61 bis 66 für die Kommunikation mit einem Datenverwaltungssystem zur Verfügung gestellt. Typischerweise werden bei einer derartigen Konfiguration drei Datenverwaltungssysteme 71, 72, und 73 eingesetzt.

Es besteht keine direkte Verbindung unter den einzelnen Applikationen 11, 14 und 15. Für den Zugriff der Audiometrieapplikation 11 auf ein Noah-Datenverwaltungssystem 71 steht ein Noah-Interface für Audiometrie 61 zur Verfügung. Ein ASCII-Interface für Audiometrie 62 dient zum Zugriff der Audiometrieapplikation 11 auf ein ASCII-Datenverwaltungs-System 73. In ähnlicher Weise besitzt die Einstell- oder Fittingapplikation 14 über ein Noah-Interface für die Einstellung 63 Zugriff auf das Noah-Datenverwaltungssystem 71. Über ein IDAPI-Interface für die Einstellung 64 besteht Verbindung von der Fittingapplikation 14 zu einem IDAPI-Datenverwal-tungssystem 72. Darüber hinaus verbindet ein IDAPI-Interface für die Testbox 65 die Testboxmessungsapplikation 15 mit dem IDAPI-Datenverwaltungssystem 72. Schließlich verbindet ein ASCII-Interface für die Testbox 66 die Testboxmessungsapplikation 15 mit dem ASCII-Datenverwaltungssystem 73. Diese Interfaceverbindungen stellen lediglich eine Auswahl dar und können je nach Bedarf ergänzt werden. So kann beispielsweise auch für die Audiometrieapplikation 11 ein IDAPI-Interface vorgesehen werden.

Zum Datenaustausch beispielsweise zwischen der Audiometrieapplikation 11 und der Fittingapplikation 14 sind zwei Interfaces notwendig, die eine Verbindung zu einem gemeinsamen Datenverwaltungssystem herstellen. Im vorliegenden Fall gemäß FIG 2 sind dies die beiden Noah-Interfaces 61 und 63. Die Testboxmessungsapplikation 15 kommuniziert mit der Audiometrieapplikation 11 beispielsweise über die beiden ASCII-Interfaces 62 und 66 und dem gemeinsamen ASCII-Datenverwaltungssystem 73.

Erfindungsgemäß wird die Vielzahl von Interfaces 61 bis 66 durch ein einziges Interface 69 ersetzt, wie dies in dem Blockschaltbild gemäß FIG 3 angedeutet ist. Dieses Interface 69 umfasst die folgenden Komponenten beziehungsweise Merkmale:
- Klassenframework, das eine Klassenbibliothek darstellt. Mit ihm ist es möglich, dass das Interface 69 mit Klassenarchitektur in die Applikationen eingreift.
- Konvertierroutinen, die die entsprechenden Datenkonvertierungen vornehmen, da beispielsweise die Audiometriedaten in jeder Datenbank eine andere Struktur besitzen.
- Uniformes Interface für Applikationen, das eine einheitliche Anbindung des Interface 69 an die verschiedenen Applikationen 11, 14 und 15 ermöglicht.
- Menge von Interfaces für verschiedene Datenverwaltungssysteme. Diese sind notwendig, um den Datenbankdaten für die einzelnen Datenverwaltungssysteme 71 bis 73 die jeweils spezifische Anbindung zu geben.
- "State sharing" und "state and data notifying" über Applikationen hinweg. Damit wird gewährleistet, dass sich zwei Applikationen einen Zustand, d.h. Daten, teilen, und gegebenenfalls in einer Datenbank abspeichern.
- Datenhaltungsalgorithmen. Sie bieten die Möglichkeit, Daten dort abzulegen, wo sie am nächsten zum Datenverwaltungssystem sind. In einem flüchtigen Speicher werden die Daten beispielsweise für einen raschen Zugriff zwischengespeichert.

Die genannten Komponenten und Merkmale bieten für das universelle Interface 69 die Möglichkeit, mit einem einzigen Interface Daten in unterschiedliche Datenverwaltungssysteme zu speichern und untereinander auszutauschen. Jede Applikation kann die Klassenbibliothek verwenden und erhält damit einheitlich Zugriff auf das gemeinsame Interface 69. Folglich kann jede Applikation Daten in standardisierter Form abgeben und empfangen. Das Framework 69 beinhaltet unter anderem Objekte, die dem Anwender Datenverwaltung ermöglichen.

## Patentansprüche

1. Schnittstellenvorrichtung (69) für audiologische Geräte (1, 4, 5) zwischen mehreren Applikationen (11, 14, 15) einerseits und mindestens einem Datenverwaltungssystem (71, 72, 73) andererseits mit
einer Applikationsanschlusseinrichtung, an die die mehreren Applikationen (11, 14, 15) zum uniformen Datenaustausch anschließbar sind,
einer Datenverwaltungsanschlusseinrichtung, an die das mindestes eine Datenverwaltungssystem (71, 72, 73) zum Datenaustausch anschließbar ist, und
einer Konvertiereinrichtung, die zwischen die Applikationsanschlusseinrichtung und die Datenverwaltungsanschlusseinrichtung geschlossen ist, zum Konvertieren von von der Applikationsanschlusseinrichtung aufgenommenen für die mehreren Applikationen jeweils spezifischen Applikationsdaten in ein vorgebbares Datenbankformat und/oder zum Konvertieren von von der Datenverwaltungsanschlusseinrichtung aufgenommenen Datenbankdaten in ein oder mehrere für die mehreren Applikationen (11, 14, 15) jeweils spezifischen Applikationsformate.

2. Vorrichtung nach Anspruch 1, die eine Klassenbibliothek aufweist, auf die mit jeder der mehreren Applikationen (11, 14, 15) zugreifbar ist.

3. Vorrichtung nach Anspruch 1 oder 2, die eine Zustandsverwaltungseinrichtung für die mehreren Applikationen aufweist, so dass die mehreren Applikationen (11, 14, 15) auf vorgegebene Daten gemeinsamen Zugriff haben.

4. Vorrichtung nach Anspruch 3, wobei durch die Zustandsverwaltungseinrichtung Zustände und Daten der mehreren Applikationen (11, 14, 15) in einer Datenbank zum gemeinsamen Zugriff abspeicherbar sind.

5. Vorrichtung nach Anspruch 3 oder 4, wobei mit der Zustandsverwaltungseinrichtung automatisch erkennbar ist, welches oder welche Datenverwaltungssysteme (71, 72, 73) an die Vorrichtung (69) angeschlossen sind.

6. Vorrichtung nach einem der Ansprüche 1 bis 5, die eine Datenhaltungseinrichtung zum Halten von Daten für mehrere der Applikationen (11, 14, 15) umfasst.

7. Vorrichtung nach Anspruch 6, wobei die Datenhaltungseinrichtung einen flüchtigen Speicher aufweist.

8. Verfahren zum Datenaustausch für audiologische Geräte 1, 4, 5)zwischen mehreren Applikationen (11, 14, 15) einerseits und mindestens einem Datenverwaltungssystem (71, 72, 73) andererseits durch
uniformen Datenaustausch von Applikationsdaten mit mehreren der Applikationen durch eine Schnittstellenvorrichtung,
Datenaustausch von Datenbankdaten mit dem mindestens einen Datenverwaltungssystem durch die Schnittstellenvorrichtung, und
Konvertieren der für die mehreren Applikationsdaten jeweils spezifischen Applikationsdaten in ein vorgebbares Datenbankformat für das mindestens eine Datenverwaltungssystem und/oder Konvertieren von Datenbankdaten in ein oder mehrere für die mehreren Applikationen (11, 14, 15) jeweils spezifischen Applikationsformate.

9. Verfahren nach Anspruch 8, wobei der uniforme Datenaustausch durch eine Klassenbibliothek ermöglicht wird, auf die mit jeder der mehreren Applikationen (11, 14, 15) zugegriffen wird.

10. Verfahren nach Anspruch 8 oder 9, wobei die mehreren Applikationen (11, 14, 15) auf vorgegebene Daten gemeinsamen Zugriff haben.

11. Verfahren nach Anspruch 10, wobei Zustände und Daten der mehreren Applikationen (11, 14, 15) in einer Datenbank zum gemeinsamen Zugriff abgespeichert werden.

12. Verfahren nach einem der Ansprüche 8 bis 11, wobei automatisch erkannt wird, welches oder welche der Datenverwaltungssysteme (71, 72, 73) angeschlossen sind.

13. Verfahren nach einem der Ansprüche 8 bis 12, wobei Daten für mehrere der Applikationen (11, 14, 15) intern, insbesondere flüchtig gehalten werden.
